Europäisches Patentamt

European Patent Office

Office européen des brevets

(19)

(11) Publication number: **0 301 583 B1**

(12) **EUROPEAN PATENT SPECIFICATION**

(45) Date of publication of patent specification: **27.04.94**   (51) Int. Cl.5: **G01N 35/02**

(21) Application number: **88112355.8**

(22) Date of filing: **29.07.88**

(54) **Automatic immunological analysing apparatus.**

(30) Priority: **31.07.87 JP 190214/87**
**28.08.87 JP 212720/87**

(43) Date of publication of application:
**01.02.89 Bulletin 89/05**

(45) Publication of the grant of the patent:
**27.04.94 Bulletin 94/17**

(84) Designated Contracting States:
**CH DE FR GB IT LI NL**

(56) References cited:
EP-A- 0 051 496      FR-A- 2 357 881
FR-A- 2 373 065      FR-A- 2 390 720
GB-A- 2 130 367      GB-A- 2 131 168
US-A- 3 636 777

(73) Proprietor: **FUJIREBIO KABUSHIKI KAISHA**
**6-7, Shimo-ochiai 4-chome**
**Shinjuku-ku**
**Tokyo(JP)**

(72) Inventor: **Saito, Tomo**
**c/o Fujirebio Kabushiki Kaisha**
**6-7 Shimo-ochiai**
**4-chome Shinjuku-ku Tokyo(JP)**
Inventor: **Sakurabayashi, Yasusuke**
**c/o Fujirebio Kabushiki Kaisha**

**6-7 Shimo-ochiai**
**4-chome Shinjuku-ku Tokyo(JP)**
Inventor: **Hikita, Akio**
**c/o Fujirebio Kabushiki Kaisha**
**6-7 Shimo-ochiai**
**4-chome Shinjuku-ku Tokyo(JP)**
Inventor: **Inouchi, Toshitsugu**
**c/o Fujirebio Kabushiki Kaisha**
**6-7 Shimo-ochiai**
**4-chome Shinjuku-ku Tokyo(JP)**
Inventor: **Sone, Takashi**
**c/o Fujirebio Kabushiki Kaisha**
**6-7 Shimo-ochiai**
**4-chome Shinjuku-ku Tokyo(JP)**
Inventor: **Suzuki, Norihiro**
**c/o Fujirebio Kabushiki Kaisha**
**6-7 Shimo-ochiai**
**4-chome Shinjuku-ku Tokyo(JP)**
Inventor: **Hirakawa, Atsuko**
**c/o Fujirebio Kabushiki Kaisha**
**6-7 Shimo-ochiai**
**4-chome Shinjuku-ku Tokyo(JP)**
Inventor: **Kikuchi, Yousuke**
**c/o Fujirebio Kabushiki Kaisha**
**6-7 Shimo-ochiai**
**4-chome Shinjuku-ku Tokyo(JP)**

EP 0 301 583 B1

(74) Representative: **Barz, Peter, Dr. et al**
**Patentanwälte Dipl.-Ing. G. Dannenberg**
**Dr. P. Weinhold, Dr. D. Gudel**
**Dipl.-Ing. S. Schubert, Dr. P. Barz**
**Siegfriedstrasse 8**
**D-80803 München (DE)**

## Description

The present invention relates to an automatic analysis of immunological agglutination for use in clinical diagnosis and an apparatus for performing the same.

The analysis utilizing immunological agglutinating reaction has been used popularly in clinical test due to its advantages in selectivity and high sensitivity. A classical example thereof is the identification of blood type based on agglutination of red corpuscles and a recent example is the measurement of antigen and/or antibody related to microorganism or cancer based on a utilization of new carriers.

Examples of the apparatus which can automate such analysis or measurement are disclosed in FR-A-2 357 881, US-A-3 636 777 and JP-A-146044/1980, 111447/1982, 11858/1983, 22956/1983 and 105065/1983. In these prior arts, a series of operations based on the immunological agglutination reaction are automated to determine whether or not there is immunological reaction by optically analysing an agglutinated image. In each of these prior arts, however, a reference value of agglutination is obtained by measuring ratio of light amount passed through or reflected from a center portion of a conical container to that related to a peripheral portion thereof. Therefore, it is difficult to apply them to judgement of various agglutination images with high accuracy.

That is, although the conventional judgement which uses permeable light usually, a solution for agglutination reaction contains, in addition to substance to be detected, various proteins which may be precipitated separately from a result of the aimed agglutination reaction causes a whole of the solution to be white by which the accuracy of measurement is degraded. Such phenomenon is usually caused by a complicated combination of an aimed substance and a test solution and, particularly, it becomes severe when a long time lapses after it is prepared by mixing the substance with the test solution or the solution contains proteins and/or salts at high concentration. Further, since, in the conventional system, an amount of light passed through the solution is considerably reduced by scattering thereof by those substances precipitated, the light amount obtained does usually not always correspond to an agglutination reaction. Therefore, when the judgement is to be performed by converting concentration into luminance, it is impossible to judge an agglutination exactly.

Further, in the conventional optical detecting device for use in the judgement of agglutination, a reaction vessel or container is measured one by one. Therefore, it takes considerable time and requires a transportation device for moving microplates.

In the clinical test field, there has been no real automatic apparatus for agglutinative immuno-analysis although there has been semiautomatic apparatus in which an operation is partially automated while important steps such as test sample pouring, transportation of the test samples from a retreatment device to a detection device and a judging steps are performed manually.

An object of the present invention is to provide a fully automatic immunological agglutination analyzing apparatus which is capable of providing a highly precise, high speed analysis of immunological agglutination.

The apparatus according to the present invention is as defined in claim 1.

The pretreatment device of the apparatus according to the present invention includes a plurality of reaction containers each taking the form of a microplate having a plurality of wells, which are stacked in a container supply device. One of the microplates which is the lowest in the container supply device is derived and transported therefrom. The test sample pouring device may be one capable of drawing up a certain amount of test sample and pouring it to each of the wells of the microplate derived from the container supply device by an amout of 5 to 200 $\mu\ell$. It is preferable that the pouring device is washable or exchangeable after the pouring operation of one test sample to avoid a contamination of another test sample by the one sample. A pouring portion of the pouring device is preferably movable three-dimensionally. A reagent supply device for supplying reagent to the wells of the microplate to which the test sample has been supplied includes a plurality of reagent pouring devices, a dilution device, a washing device for washing the dilution device, a washing liquid tank and a transportation device. The reagent pouring device includes a plurality of storage tanks each capable of storing a different reagent of around 200 m$\ell$ and a plurality of pouring nozzles for supplying the reagents simultaneously to the wells. One of the reagent pouring devices supplies a diluting liquid to one of the wells by an amount of 5 to 200 $\mu\ell$ and dilutes the test sample in the well by drawing up and discharging the liquid in the well repeatedly. Another of the reagent pouring devices pours an immunological agglutination reagent to each well by an amount of 5 to 200 $\mu\ell$. The microplate having the wells each filled with the test sample and the immunological agglutination reagent is transported to an agitation device of vibration type which eliminate undesired vibration of other portions of the apparatus.

After the agitation, the microplate is transported to the detection device. The detection device comprises an image pick-up device, a transportation device and a container collecting device. The image pick-up device includes an image input portion, an image memory, an image output portion, a camera and a suitable light source. The camera is, in this invention, a TV camera such as CCD or solid state image tube. However, the camera may be any visual sensor although TV camera is preferable in processing image data. Light from the light source passes through agglutination images of all of the wells of the microplate and it containing the agglutination image information is inputted to the TV camera by which the agglutination in the wells of the microplate is picked-up. That is, agglutinated images in the wells of the microplate is illuminated by light from the light source and lights passed through the respective wells are picked-up by the TV camera as optical informations. The light source used in this invention is enough to illuminate the all of the wells of the microplate uniformly. The optical information is supplied to the image input portion and stored in the image memory portion which is derived by the image output portion according to demand.

In the present invention, the agglutination images can be picked up successively after a commencement of agglutination reaction or once after the agglutination reaction completes. In order to process the microplates which are transported successively to thereby perform either of the above mentioned image pick-up operations, it is preferable that the microplates are put in a detection position sequentially by the container transportation device which may be of a turn-table type whose rotation may be intermittent interval of which is controllable. When the final image is to be picked up, the container may be transported to the detection position after 90 to 120 minutes from a completion of the agitation.

After the agglutination image information of one microplate, it is transported by the same container transportation device to the container collecting device.

The judgement of agglutination is performed according to the technique entitled "Method and Apparatus for Judging Agglutination Pattern" disclosed in EP-A-257660 of Applicant.

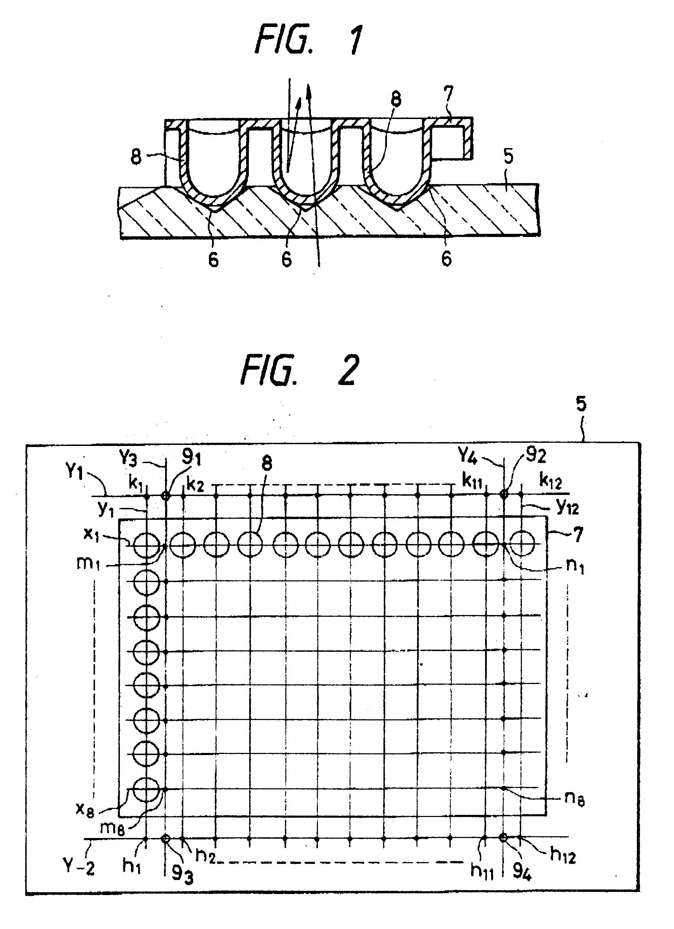

This will be described in brief with reference to Figs. 1 to 5. In these figures, a plate stand 5 is formed of a transparent plastic material, and its upper surface has, as shown in Fig. 1, substantially conical recesses 6 for receiving wells 8 of the microplate 7 at precise positions. Positioning openings 9 are perforated at the positions corresponding to vertexes of a rectangle and light passes through the plate stand 5 vertically upwardly. A main light source (not shown) is provided above the plate stand 5. An auxiliary light source of less power than the main light source may be provided below the plate stand 5. The coordinates of the positioning openings 9 are used to determine the center position of each well. In Fig. 2, it is assumed that the positions of the positioning openings are at coordinates of $9_1$ $(x_1,y_1)$, $9_2(x_2,y_2)..9_4(x_4,y_4)$. Then a line connecting the openings 9 and 9 is expressed by $Y_1 = a_1 x + b_1$. Similarly, three linear lines $Y_2$, $Y_3$ and $Y_4$ connecting the openings $9_3$ and $9_4$, $9_1$ and $9_3$ and $9_2$ and $9_4$, respectively, are obtained.

Then, each of a total of four linear lines is divided into 11 equal segments to obtain 12 points $k_1$,.., $k_{12}$, $h_1$,.., $h_{12}$, $m_1$,..$m_8$ and $n_1$,.., $n_8$ are obtained, respectively. Then, 12 lines $y_1$,.., $y_{12}$ connecting horizontally these points ki and hi and 8 linear lines $x_1$,.., $x_8$ connecting the points mi and ni are obtained. Then, the cross points of the linear lines yi and xi are obtained, which are used as the centers of the wells.

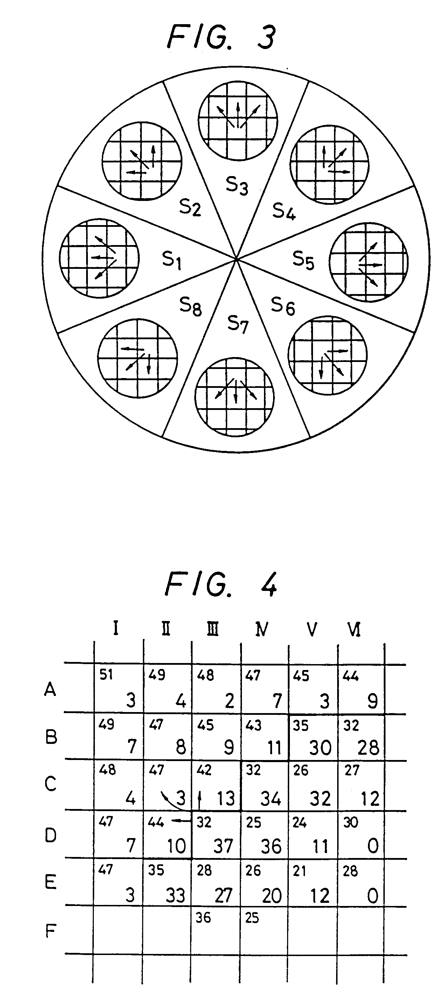

Then, as shown in Fig. 3, an image of each well is divided radially into 8 sectors $S_1$, $S_2$,.., $S_8$, and the differential values of the pixels in the sectors $S_1$ to $S_8$ are determined, respectively. The differential value of each pixel is a total of difference in luminance between the pixel and adjacent three respective pixels disposed radially outwardly of the pixel in question. In the sector $S_1$, pixels disposed adjacent to the left side, oblique upper left side and oblique lower left side of a pixel are to be compared in luminance with the latter and a total of differences are to be determined as the differential value of the pixel in question. In the sector $S_2$, pixels disposed adjacent to the upper side, left side and oblique upper left side of a pixel are compared in luminance with the latter and a total of differences are to be determined as a differential value thereof, and so on. Fig. 4 shows the differential values of the pixels in the sector $S_2$. When the differential value of the pixel D-III having an intensity of "32" is considered, as an example, a differential value of this pixel is 37 because intensities of pixels D-II, C-II and C-III outwardly surrounding the pixel D-III are respectively "44", "47" and "42", i.e., 12(= 44-32) + 15(= 47-32) + 10(= 42-32) = 37. Thus, the differential value of each pixel is determined.

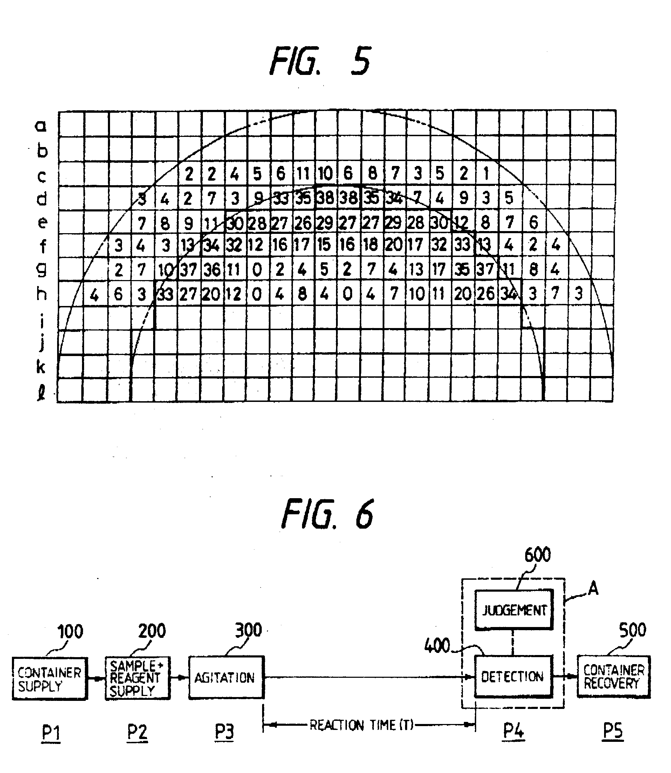

When the differential values of the respective pixels are determined, the values of the pixels aligned on one line are compared with each other to detect two pixels having largest two values in the same line as defining the edge of the agglutination. That is, when differential values are given as shown in Fig. 5, two pixels in, for example, lines which have largest value 38 are determined as defining the edge and, in line f, the pixels having values 34 and 33 are detected as defining the edge. Similarly, when the edges are selected for all the lines, the entire edge becomes as designated by a thick solid line as shown in Fig. 5 in which dotted chain lines indicate the actual edges of the agglutination portion. The number of the pixels inside the edge are counted, and the area of the agglutination is compared with a reference value. The contour of the agglutination is defined clearly by calculating the variation coefficient of the differential value and the contrast of the image is obtained by averaging the differential values.

When final agglutination images belong to a certain kind, the values obtained as above mentioned are used as they are and, when there are series of images belonging to different kinds, a variation rate thereof is obtained.

The distribution of pixels having luminance level other than zero is evaluated by means of a microcomputer. A standard deviation of the distribution is obtained and is divided by a mean luminance level to obtain a variation coefficient CV. Since the CV does not contain factors due to variation of contrast, the problem caused thereby can be completely removed. The CV values obtained in this manner are plotted in the area (number of the pixels) to obtain a distribution for special item, the judged results are superposed on the CV distribution to determine the boundary of the judgement, which is stored in a computer memory.

Simultaneously, the same agglutination images are judged by skilled visual judge personnel and results are also plotted. On the latter plot, a linear line is set empirically to divide it to a region which is decided by the skilled personnel as positive and the other region which is decided as negative. The linear line is used as a reference line which is memorized.

The judgement of the test sample is performed with reference to this reference line.

The above mentioned judging system is well automated as disclosed in EP-A-257660.

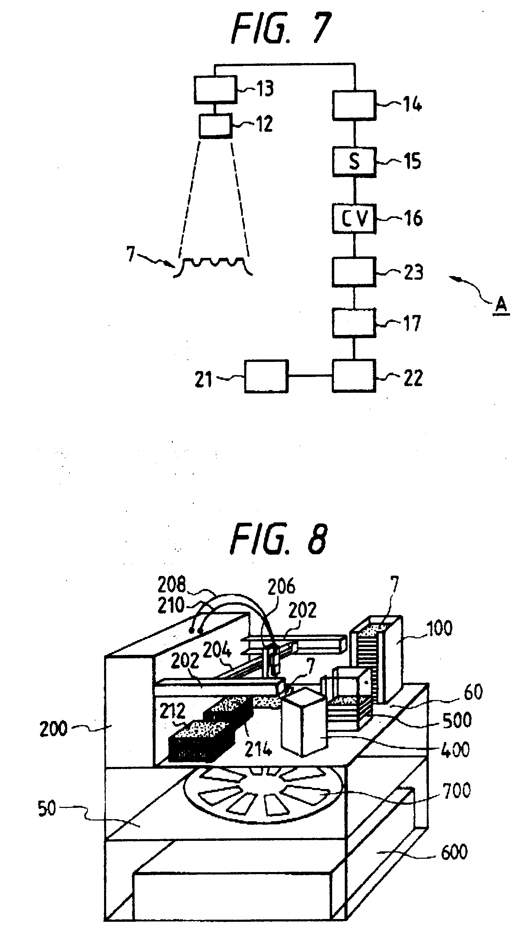

Figs. 6 and 7 show examples of the system for performing the above mentioned operation with reference to Figs. 1 to 5, respectively. In Fig. 6, an agglutination image on a microplate 7 is stored as a video image in a TV camera 12 in an image memory 13, and the stored image is differentiated and, then, an area of the image is obtained by an image processor 15.

Standard deviation S of the pixel having intensity level not higher than zero is obtained by a standard deviation calculator 16 from the obtained profile and area of the image, and its CV is further obtained by a CV calculator 17. The CV is supplied to a plotter 18 together with the area obtained by the image processor 15, and is plotted on a graph having, for example, an ordinate as CV and abscissa as the area.
In the drawing:

Figs. 1 to 5 are explanatory illustrations for explaining a principle of contour derivation of agglutination image which can be used in the present invention effectively;

Fig. 6 shows a flow of a reaction container in an apparatus for performing the present invention;

Fig. 7 is a block diagram of a detection/judgement device suitable to use in the present apparatus;

Fig. 8 is a perspective view of an embodiment of an automatic agglutination judging apparatus according to the present invention;

Fig. 9 is a plan view of the apparatus shown in Fig. 8;

Fig. 10 is a side view of the an automatic agglutination judging apparatus shown in Fig. 8;

Fig. 11 shows, schematically, an agitation device suitable to use in the embodiment shown in Fig. 8;

Fig. 12 shows another embodiment of the agitation device;

Fig. 13 is a front view of another embodiment of the present apparatus, with a front wall portion thereof being removed;

Fig. 14 is a plan view of the embodiment shown in Fig. 13;

Fig. 15 is a side view of the apparatus shown in Fig. 13;

Fig. 16 is a plane view of a reaction container transportation device of the embodiment shown in Fig. 13;

Fig. 17a is a plan view of a container supply device of the apparatus shown in Fig. 13;

Fig. 17b is a partially cross sectioned side view of a bottom portion of the container supply device;

Fig. 18a is a plan view of a container recovery device of the apparatus in Fig. 13;

Fig. 18b is a partially cross sectioned side view of the container recovery device;

Fig. 19 shows, schematically, a vertical transportation device for transporting a container to a position in which a pouring operation thereto is performed;

Fig. 20 is a front view of another embodiment of the agitation device;

Fig. 21 shows a portion of another embodiment of the agitation device; and

Figs. 22 to 24 are graphs showing references of judgement for ATLA.

In Fig. 6 which shows a flow of a reaction container, i.e., a microplate having a plurality of wells in an automatic immunological agglutination judgement apparatus for performing the present method, the reaction container is supplied from a container supply device 100 which is conveyed by an intermittent transportation device which is to be described later to a first position P1 in which a sample and a suitable reagent are poured by a sample/reagent pouring device 200 and, after a sample and reagent are supplied to each of the wells of the microplate, the microplate is conveyed to a second position P2 in which it is vibrated by an agitation device 300 to agitate the contents of the wells to thereby mix them in each well. After the agitation is completed, the microplate is transported by the same transportation device over a substantial distance long enough to give a sufficient reaction time T to a third position P3 in which a resultant agglutination is detected by a detection device 400. Then, the microplate is transported to a fourth position P4 in which it is recovered by a container recovery device 500. An output of the detection device 400 is supplied to a judging device 600.

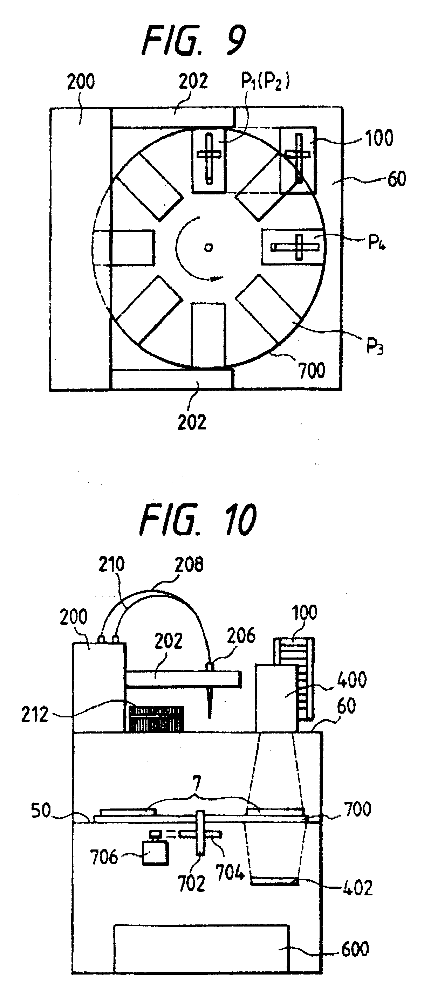

An example of a construction of a combined detection device/judging device A shown by a dotted line in Fig. 6 is shown in block in Fig. 7, although either of the constructions shown in Figs. 6 and 9 of the aforesaid EP-A-257660 may be used if necessary. In Fig. 7, an agglutination image of wells of a microplate 7 is picked up by a TV camera 12 of the detection device 400 which further includes an image memory 13 and is stored as a video image in the image memory 13 which is supplied to the judging device 600 comprising a image processor 14, a standard deviation calculator 15, a variation coefficient calculation device 16, a plotter 17, a memory 21 storing a boundary line obtained by skilled personnel and a comparator 22 for compring an output of the plotter 17 with the content of the memory. If necessary, the judging device 600 may further include a contrast calculator 23 disposed between the variation coefficient calculator 16 and the plotter 17.

An embodiment of the present apparatus is shown in Figs. 8 to 11, in detail. In Fig. 8 which is a perspective schematical illustration of the present apparatus, Fig. 9 which is a top view of the apparatus and Fig. 10 which is a side view thereof, a transportation device 700 in the form of rotary disc is provided on a support frame 50 below which the judging device 600 is arranged. On the transportation device 700, a plurality of microplates 7 can be received equiangularly. The microplates 7 are supplied from the container supply device 100 onto the transportation device 700 one by one while the latter is rotated by a suitable driving means 706 through a suitable transmission gear mechanism including a gear 704 mounted on a rotary shaft 702 of the transportation device 700. The container supply device 100 will be described in detail with reference to another embodiment of the apparatus.The microplate 7 fed from the container supply 100 onto the transportation device 700 is firstly carried to a sample/reagent supply position P1 thereby at which the sample and the reagent are poured to respective wells of the microplate 7 by means of a pouring nozzle member supported three dimensionally movably by a vertically movable support 206 which is movable in a horizontal plane. That is, the support 206 is supported by a lateral guide member 204 which, in turn, is supported by a pair of guide members 202 extending from a pouring device 200. A suction tube 208 is connected to the nozzle member to suck a predetermined volume of the sample from a sample holder 212 and to discharge it to the wells of the microplate 7 in the position P1. The nozzle member is changed sample by sample. New nozzles are picked up from a chip holder 214. The position of the nozzle is detected by a suitable detector connected through a lead wire 210 to a control portion of the pouring device 200 to control it suitably.

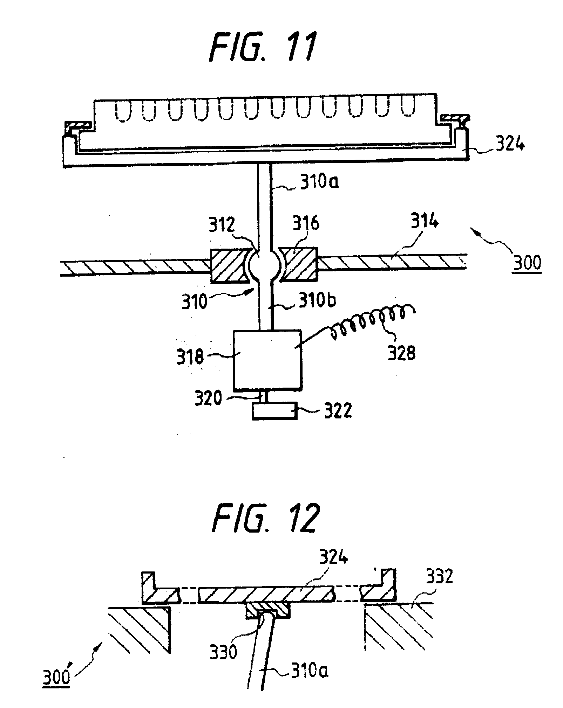

After the pouring operation is completed, the microplate is carried with intermittent rotation of the transportation device 700 to a position P2 at which the microplate 7 is vibrated to mix the sample and the reagent in the respective wells thereof. Although, in Fig. 9, the positions P1 and P2 are overlapped, the position P2 can be set at a next position to the position P1. The vibration of the microplate 7 is achieved by an agitation device 300 such as shown in Fig. 11.

In Fig. 11, a rod 310 has a spherical portion 312 in a center portion thereof, which is supported by a universal bearing 316 having a corresponding shape. A motor 318 is fixedly mounted on a lower end 310b of the rod 310 and an eccentric weight 322 is mounted on an output shaft 320 of the motor 318.

A plate member 324 is fixed to an upper end 310a of the rod 310 on which the microplate 7 is suitably mounted fixedly. The motor 318 is connected through flexible lead wires 328 to a control/power source which are not shown. With rotation of the motor 318, the eccentric weight 322 is rotated by which a revolution momentum is produced on the output shaft 320 of the motor 318. Therefore, the rod 310 revolutes around the universal bearing as a fulcrum, so that the upper end 310a as well as the plate member 324 fixed thereon revolute similarly causing revolution of liquid in each of the wells of the microplate 7.

The radius and speed of the revolution of the rod 310 are determined by rotation speed of the motor 318, eccentricity of the eccentric weight 322, length of the rod 310 and position of the universal bearing. When the eccentricity, the rod length and the position of the universal bearing are fixed, the higher the motor speed provides the larger the radius and the higher the revolution speed. Theoretically, it is possible to make a vibration component given to the support structure of the apparatus zero by suitably balancing upper and lower movable portions of this agitation device with respect to the universal bearing. It should be noted in this embodiment of the agitation device that there is an overflow problem of liquid in the wells due to inclination of the plate member 324 when the revolution radius is too large.

Fig. 12 shows another embodiment of the agitation device 300, by which the problem of overflow is solved. In Fig. 12, the plate member 324 is not fixed to the upper end 310a of the rod 310 but supported horizontally by a suitable support member 332. The plate member 324 is formed on a lower surface thereof a recess member 330 which receives the upper end 310a of the rod 310. With revolution of the rod 310, the plate member 324 slides horizontally on the support member 332 which may be formed integrally with the support member 314.

After the agitation is completed, the microplate 7 is transported to a position P3 in which the microplate 7 is illuminated by a light source 402 to pick up agglutination images of the respective wells thereof by means of a TV camera 400 output of which is suppied to a image processing device 600 in which the processing mentioned previously is performed.

In order to give a time T (Fig. 6) long enough to achieve immunological reaction in the wells, a distance between the positions P2 and P3 is made large. Then, the microplate 7 is transported to a position P4 in which it is recovered in a container recovery device 400 which will be descibed in detail later.

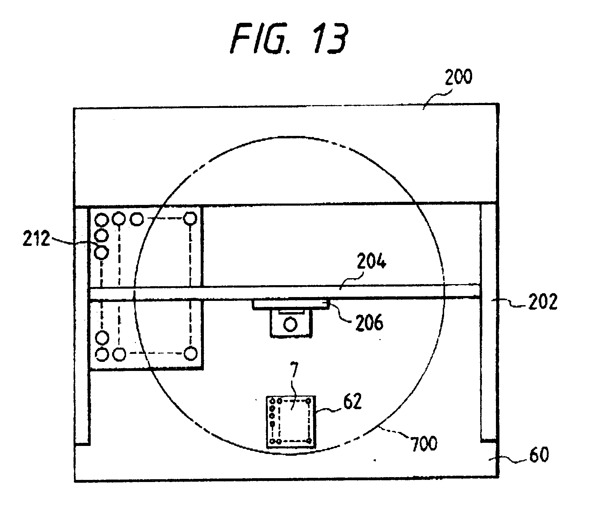

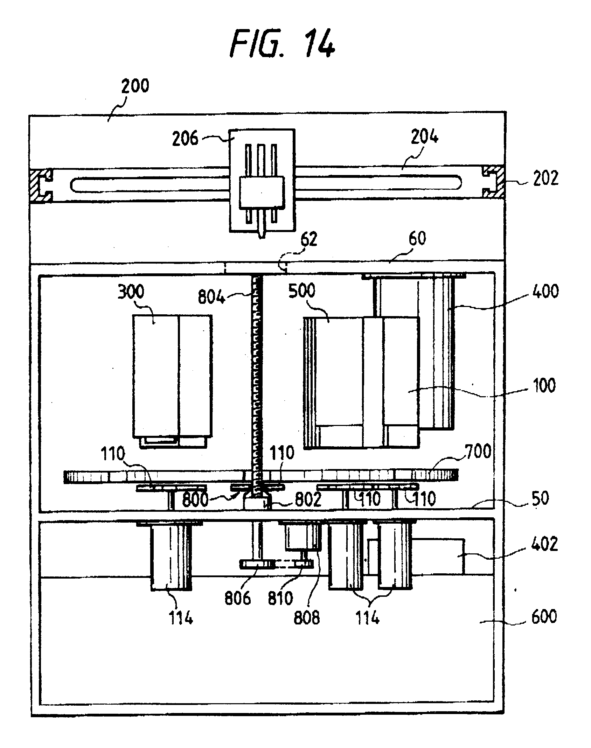

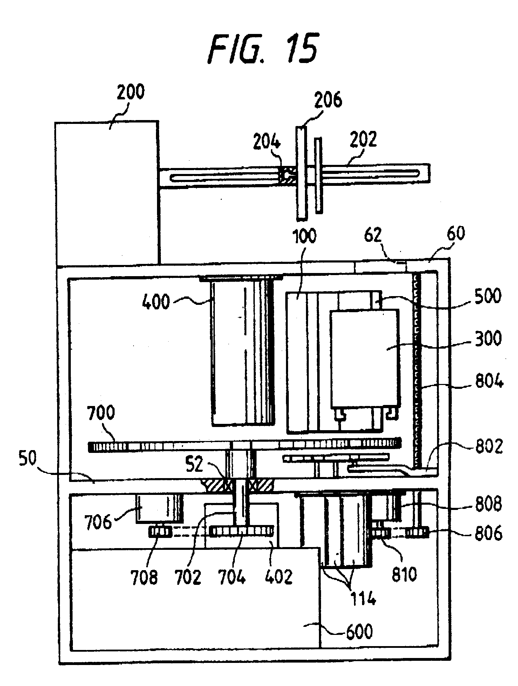

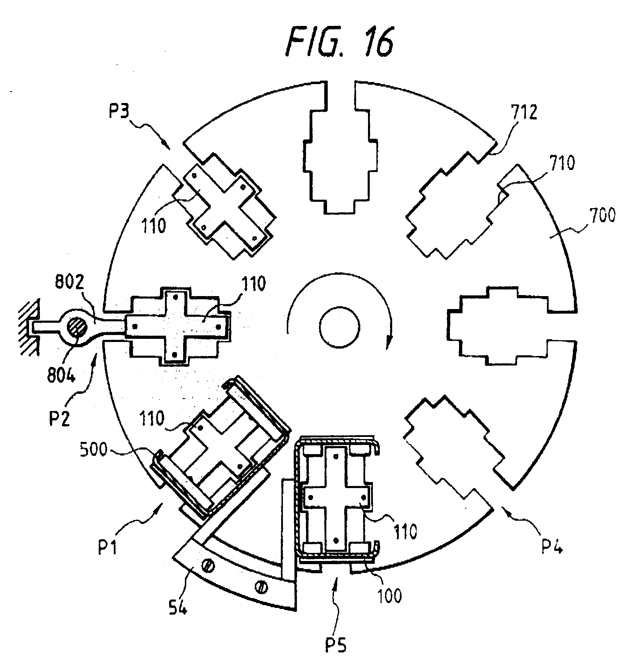

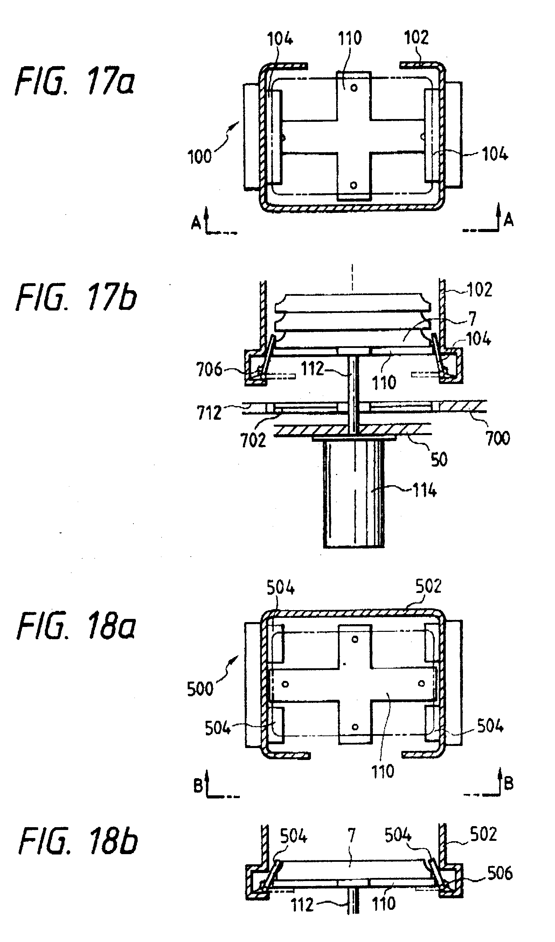

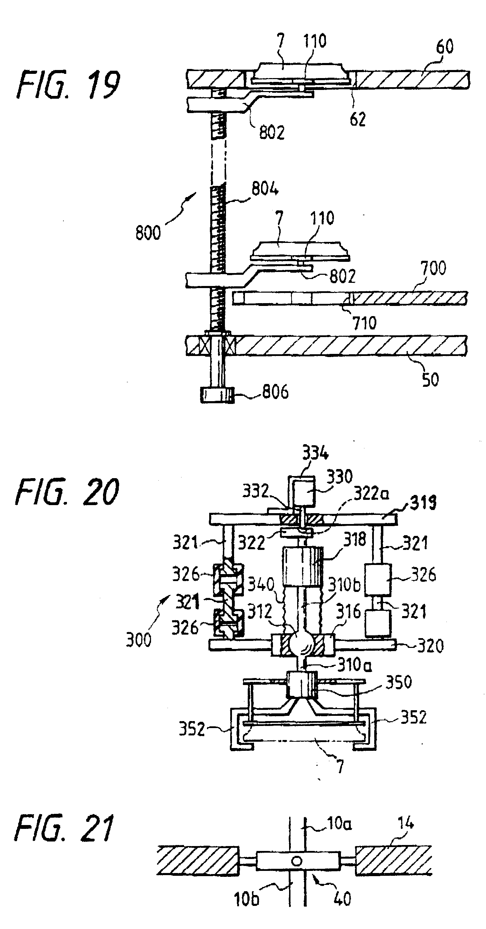

Fig. 13 is a plan view of another embodiment of the present apparatus in which the constitutional components are moved onto a middle deck 50 so that only the pouring operation is performed on a top deck 60. The embodiment will be described in detail with reference to Fig. 14 which is a front view thereof, Fig. 15 which is a side view thereof, Fig. 16 which shows a transportation device 700 in detail, Figs. 17a and 17b which show a container supply device 100, Figs. 18a and 18b which show a container recovery device 400, Fig. 19 which shows a device 800 for moving the microplate 7 to a pouring position and Fig. 20 which shows a agitation device 300.

In Fig. 13, the top deck 60 is formed with an opening 62 through which a microplate 7 lifted up from the transportation device 700 by the elevation device 800 in the pouring position is exposed for pouring operation. The elevation device 800 is composed of a vertical lead screw 804 rotatably supported between the top and the middle decks 60 and 50 and a support member 110 in the form of cross plate having an arm an end portion 802 of which meshes with the lead screw 804 as best shown in Figs. 16 and 19. The container supply device 100 and the container recovery device 400 are supported by a support structure 54 fixed on the middle deck 50 as shown in Fig. 16. The agitation device 300 is suitably supported above the transportation device 700 by a structural frame and a TV camera 400 may be supportd by the top deck 60 as shown.

The transportation device 700 in the form of rotary disc is rotatably supported by a shaft 702 supported by a bearing 52 supported by the middle deck 50. A gear 704 is fixedly mounted on a lower end of the shaft 702 which meshes with a gear 708 mounted on an output shaft of a motor 706 fixed on a lower surface of the middle deck 50 through a suitable transmission mechanism so that the disc 700 is rotated stepwise. The motor 706 may be a step motor. The disc 700 is formed with a plurality of generally rectangular openings 710 peripherally. Sides of each opening 710 are recessed respectively to allow a cross shaped elevation member 110 to pass through and an outer one of the sides is notched as shown by a numeral 712 to allow the arm of the elevation device 800 to pass through.

The container supply device 100 comprises a generally rectangular cylinder shaped cover frame 102 having one side opened as shown in Fig. 17a. A plurality of microplates 7 are stacked therein and the lowest microplate 7 is supported by a pair of oppositely disposed flaps 104 as shown in Fig. 17b. The flaps 104 covers full width of the frame and are hinged at bottom edges of the frame 102 and biased by springs 106 such that they are kept horizontally to catch a bottom of the lowest microplate 7 and, when pushed up by the elevation member 110, turned-up to allow only the lowest microplate 7 to drop out from the frame 102. In this embodiment, the elevation member 110 is supported by a od 112 coupled to a piston of a cylinder member 114 fixed to the middle deck 50. However, any other elevation mechanism may be used instead of the piston-cylinder mechanism.

Fig. 18a shows the container recovery device 500 and Fig. 18b is a partial cross section of a bottom portion of the device 500, taken along a line B-B in Fig. 18a. The container recovery device 500 comprises, as in the case of the container supply device 100, a generally rectangular cylinder shaped frame 502 and two pairs of opposite flaps 504. Spring-biased hinge mechanism of the flaps 504 is substantially the same as that of the container supply device 100 except that corresponding flaps 504 of the respective pairs are separated from each other to allow the elevation member 110 to pass through. It should be noted that such elevation members 110 are provided in the positions P1, P2, P3 and P5, respectively.

Fig. 20 shows, in partially cross section, the agitation device 300. The agitation device 300 in Fig. 20 is fundamentally the same as that shown in Fig. 12, although inverted. The device 300 in Fig. 20 comprises a first support structure including an upper deck 319, a lower deck 320 and pillars 321 connecting the decks 319 and 320, a vibration mechanism including a motor 318 having an output shaft mounted an eccentric member 322 thereon, a rod 310 having an upper and a lower rod halves 310a and 310b and a center sphere 312 supported by a bearing 316 supported by the lower deck 320 and a second support structure for supporting a microplate catch mechanism including a pair of nails 352 and a control device 350 therefor attached to a lower end of the lower half 310a of the rod 310.

The upper deck 319 is suitably fixed to the frame structure of the apparatus and each of the pillars is composed of a pair of rod portions 321. An upper end of the upper rod portion 321 is fixed to a lower surface of the upper deck 319 and a lower end thereof is connected through a shock absorber 326 to an upper end of the lower rod portion 321 whose lower end is connected through a similar shock absorber 326 to the lower deck 320. Therefore, a transmission of vibration to the apparatus structure is prevented.

On the upper deck 319, a solenoid 330 is fixed by a support 334. A plunger 332 of the solenoid 330 can penetrate the upper deck 319 into a recess 332a formed in a portion of the eccentric member 322 corresponding to the output shaft upon energization of the solenoid 330 when the agitation device stops to operate, so that undesirable swing motion of the microplate caught by the hook nails 352 is prevented. Fig. 21 shows another embodiment of the universal bearing of the agitation device. The universal bearing takes the form of gimbal whose structure and operation are well known.

An operation of the embodiment shown in Figs. 13 through 20 will be described with reference to Fig. 16.

In the position P1, the cylinder 114 associated therewith is actuated to lift up the associated elevation member 110 through the opening 710 of the disc 700 into the container supply device 100. The elevation member 110 pushes up the lowest microplate 7 while folding the flaps 104 thereof (Figs. 17a and 17b) and carries down and leaves it on the opening 710 of the disc 700 while retreaving.

The microplate 7 on the disc 700 is then transported to the position P2 by a rotation thereof by a predetermined unit angle. At this time, the same operation is performed for a new opening 710 of the disc 700 which comes into the position P1. In the position P2, the motor 808 is actuated to rotate the lead screw 804 (Figs. 14 and 19) so that the portion of the arm 802 meshed therewith and hence the elevation member 110 is moved upwardly through the opening 710 of the disc 700 while picking the microplate 7 thereon up to ultimately position the microplate 7 in the opening 62 of the upper deck 60 of the apparatus. When the microplate 7 is fixedly positioned in the opening 62, the pouring device 200 is actuated to pour predetermined liquid to the respective wells of the microplate.

After the pouring operation is completed, the motor 808 is reversed to rotate the lead screw 804 in reverse direction to thereby lower the elevation member 110 through the opening 710 of the disc 700, so that the microplate 7 is put on the disc 700 again. Then the disc is rotated by one step to the position P3. In the position P3, the elevation member 110 associated with that position is moved up to lift the microplate having liquid filled wells up to the hook nails 352 of the agitation device 300 arranged above the position P3 with the plunger 332 being received in the recess 322a of the eccentric member 322 and, at a top position of the elevation member 110, the nail control device 350 is actuated to hold the microplate by the nails 352. When the microplate 7 is caught by the nails 352, the solenoid 330 is deenergized to make the eccentric member 322 free and then the motor 318 is actuated to agitate liquid in the respective wells.

After the agitation is completed, the elevation member 110 is lowered through the opening 710 of the disc 700 leaving the microplate thereon. Thereafter, the disc 700 is rotated by several steps to the position P4. A time period required for transportation of the microplate from the position P3 to the position P4 should be long enough to obtain a desired degree of immunological reaction. In the position P4, the microplate 7 is illuminated through the opening 710 by the light source 402 and light passed through the microplate and containing agglutination information is picked up by the TV camera 400 the output of which is processed by the processor 600 as mentioned previously.

Then, the disc 700 is rotated by one step to transport the microplate to the position P5. In the position P5, the elevation member 110 associated therewith is lifted up through the opening 710 to push the microplate thereon into the bottom of the container recovery device 500 arranged thereabove while folding the flaps 504 which are returned to the original states after the microplate passes therethrough. Then, the elevation member 110 is lowered while leaving the microplate in the recovery device, and one cycle of operation completes. The same operation is performed for the microplates successively.

A control device for controlling, in synchronism with the intermittent operation of the disc 700, the operations of the elevation member 110 associated with the container supply device 100, the pouring device 200 and the associated elevation device 800, the agitation device 300 and the associated elevation member 110, the TV camera and the associated light source 402 and the elevation member 110 associated with the container recovery device 500 may be easily constructed by those skilled in the art and, therefore, details thereof are omitted in this description.

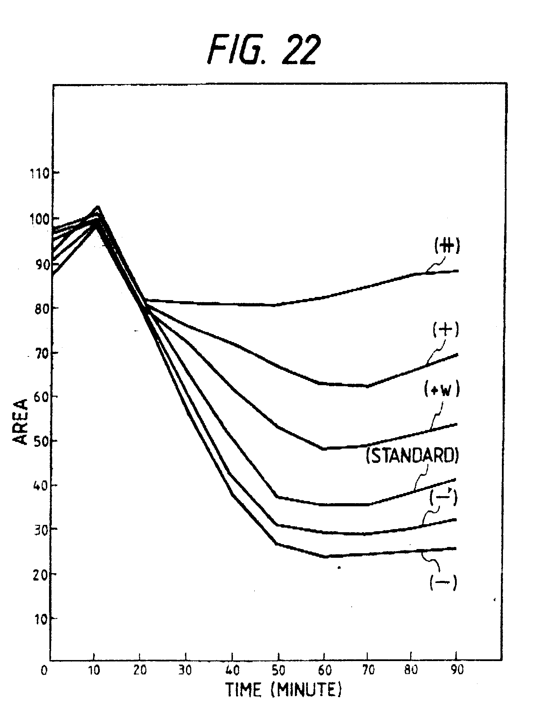

The time to be given for the travel of the container 7 on the rotary disc 700 from the position PS3 to the position PS4, which is enough to achieve the desired antigen-antibody reaction in the wells should be as short as possible in designing the present apparatus. Figs. 22 to 24 are graphs useful to determine the time, i.e., the rotational speed of the rotary disc 700.

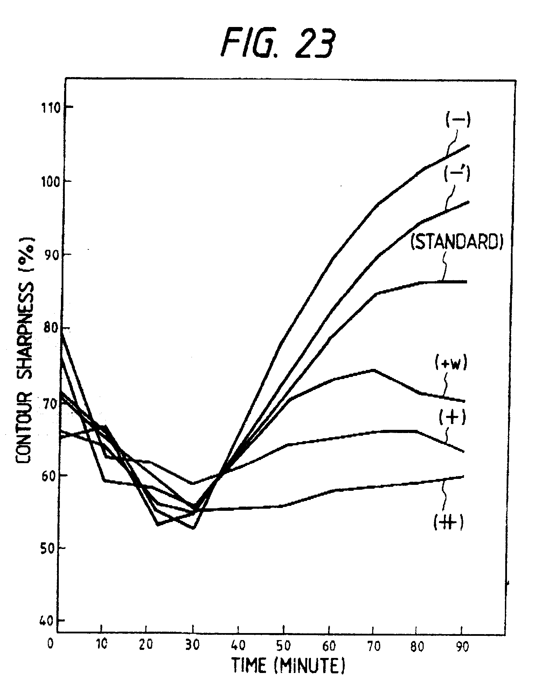

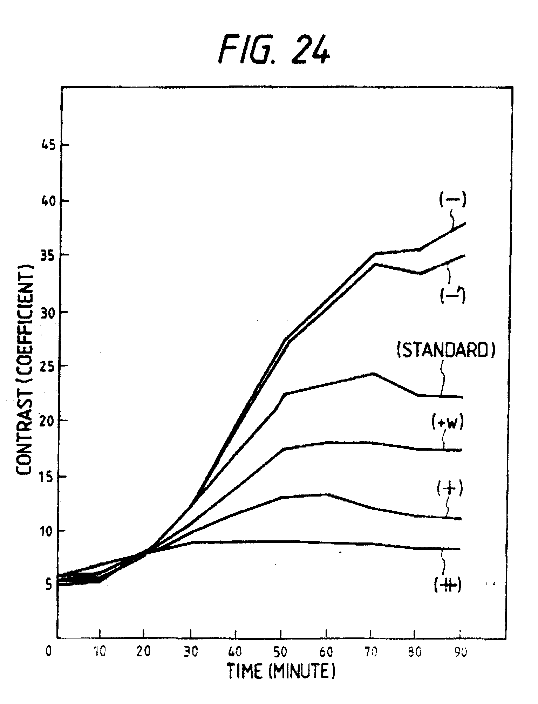

Fig. 22 is a graph showing a relation of area obtained from a contour of agglutination determined by the image processor 600 to time, Fig. 23 is a graph showing a relation of the contour of the agglutinated image to time and Fig. 24 is a grapg sowing a relation of contrast of the agglutinated image to time, for anti-adult T cell leukemia antibody as a test item. As is clear from Figs. 22 to 24, the time necessary to achieve the desired reaction can be clearly defined around 30 minutes in the case of Fig. 22, around 50 minutes in the case of Fig. 23 and around 40 minutes in the case of Fig. 24. In any of these three cases, the time is much shorter than in the conventional case which may be around 2 to 3 hours. Thus, the present apparatus should be designed such that the container 7 on the rotary disc 700 moves from the position PS3 to PS4 for about 50 minutes or less.

## Claims

1. An automatic immunological analysing apparatus comprising
   container supply means (100) for successively supplying containers (7) each having a plurality of wells,
   pre-processing means (200) for supplying agglutination immunological reagent containing antigen or antibody and samples possibly containing antibody or antigen to the wells of each container (7),
   vibrating means (300) for vibrating said container (7) to agitate the reagent and samples in said wells,
   holding means for holding said container (7) stably for a time long enough to allow an antigen-antibody reaction between the sample and the reagent in said wells to occur while transporting said container (7),
   information pick-up means (400) for obtaining agglutination pattern images of said wells of said container (7) resulting from antigen-antibody reaction,
   judging means (600) for judging the agglutination images according to information supplied from said information pick-up means (400), and
   recovery means (500) for recovering said container (7) after judgement is completed,
   characterized in that a frame structure having a top deck (60), a middle deck (50) and a bottom plate is provided, said pre-processing means (200) being disposed on said top deck (60) of said frame structure;
   that said holding means comprises a rotary disc (700) rotatably supported on said middle deck (50) of said frame structure and having a plurality of equiangularly spaced container supporting openings (710) arranged peripherally thereof, and first drive means (702, 704, 706) for rotating said rotary disc (700) intermittently from a first position (P1) to a last position (P5) so that analysis of one container (7) is completed while said container (7) is transported from said first position (P1) to said last position (P5) completing one revolution of said rotary disc (700); and
   that said vibrating means (300) comprises an electric motor (318) having an output shaft mounting thereon an eccentric weight member (322) and a rod (310) extending oppositely to said output shaft, a support structure (319,320,321) having a universal bearing (316) for supporting the center portion of said rod, and a container holding means (350,352) engageable with the free end of said rod for holding said container (7), said support structure (319,320,321) being supported by said frame structure through shock absorbing means (326) and suspending said container holding means (350,352), and means (330,332) for allowing said container holding means (350,352) to revolute only when agitation is desired which means comprises a solenoid (330) having a plunger (332) engageable with said eccentric weight member (322).

2. The apparatus claimed in claim 1 wherein said container supporting opening (710) has a size and configuration suitable to support said container (7) with a peripheral edge portion of said opening (710); said container supply means (100) is disposed above said first position (P1) of said rotary disc (700) and comprises cylinder means (102) having a cross section suitable to receive a plurality of said containers (7) in stack, flap means (104) provided in a bottom portion of said cylinder means (102) for normally closing said bottom portion of said cylinder (102) to hold said containers (7) therein, a first plate member (110) and first lift means (112, 114) for vertically moving said first plate member (110) through said container supporting opening (710) of said rotary disc (700) at said first position (P1), said first plate member (110) being adapted to open said flap means (104) and support the lower surface of the lowest container (7) in said cylinder (102) when lifted up by said first lift means (112, 114) and to carry the lowest container (7) from said cylinder (102) and put it on said container supporting opening (710) of said rotary disc (700) at said first positions (P1) when lowered by said first lift means (112, 114).

3. The apparatus claimed in claim 1 or 2 wherein a vertical means (800) is provided at a second position (P2) next to said first position (P1) for lifting said container (7) supported by said container supporting opening (710) and moved from said first position (P1) up to said pre-processing means (200) disposed on said top deck (60) of said frame structure and, after being pre-processed thereby returning said container (7) back to said container supporting opening (710).

4. The apparatus claimed in claim 3 wherein said vertical means (800) comprises a second plate member (110) and second lift means (802, 804) for vertically moving said plate member (110) through said container supporting opening (710) of said rotary disc (700) at said second position (P2).

5. The apparatus claimed in any of claims 1-4 wherein said vibrating means (300) is disposed at a third position (P3) of said rotary disc (700) and said information pick-up means (400) and said recovery means (500) are disposed in the last two positions of said rotary disc (700) in succession respectively, and wherein the peripheral rotational speed of said rotary disc (700) is determined such that sufficient time to obtain the desired degree of immunological reaction is given while rotating along a peripheral distance of said rotary disc (700) between said third position (P3) and an upstream position of said last two positions.

**Patentansprüche**

1. Automatisches immunologisches Analysegerät, umfassend
eine Behälterzufuhr-Einrichtung (100) für die aufeinanderfolgende Zufuhr von Behältern, die jeweils mehrere Vertiefungen aufweisen,
eine Vorbehandlungs-Einrichtung (200) für die Zufuhr von immunologischem Agglutinationsreagens, das Antigen oder Antikörper enthält, und von Proben, die möglicherweise Antikörper oder Antigen enthalten, in die Vertiefungen jedes Behälters (7),
eine Vibrations-Einrichtung (300) zum Vibrieren des Behälters (7), um das Reagens und die Proben in den Vertiefungen zu schütteln,
eine Halte-Einrichtung zum stabilen Halten des Behälters (7) für ausreichend lange Zeit, damit eine Antigen-Antikörper-Reaktion zwischen der Probe und dem Reagens in den Vertiefungen stattfinden kann, während der Behälter (7) transportiert wird,
eine Informationsaufnahme-Einrichtung (400) zum Erstellen von Agglutinationsmusterbildern der Vertiefungen des Behälters (7), welche bei der Antigen-Antikörper-Reaktion entstehen,
eine Auswertungs-Einrichtung (600) zum Auswerten der Agglutinationsbilder entsprechend der von der Informationsaufnahme-Einrichtung (400) zugeleiteten Information und
eine Rückführ-Einrichtung (500) zum Rückführen des Behälters (7) nach beendeter Auswertung,
dadurch gekennzeichnet, daß eine Rahmenstruktur mit einem oberen Deck (60), einem mittleren Deck (50) und einer Bodenplatte vorgesehen ist, wobei die Vorbehandlungs-Einrichtung (200) auf dem oberen Deck (60) der Rahmenstruktur angeordnet ist;
daß die Halte-Einrichtung eine Drehscheibe (700), die drehbar auf dem mittleren Deck (50) der Rahmenstruktur gehalten wird und entlang ihres Umfangs in gleichwinkeligem Abstand mehrere Behälterhalteöffnungen (710) aufweist, und eine erste Antriebseinrichtung (702,704,706) zum intermittierenden Drehen der Drehscheibe (700) von einer ersten Position (P1) zu einer letzten Position (P5) umfaßt, so daß die Analyse eines Behälters (7) vollständig ist, während der Behälter (7) von der ersten Position (P1) zu der letzten Position (P5) transportiert wird und eine Umdrehung der Drehscheibe (700) vervollständigt; und
daß die Vibrations-Einrichtung (300) umfaßt einen Elektromotor (318) mit einer Abtriebswelle, an der ein exzentrisches Gewichtselement (322) befestigt ist, und einem Stab (310), der sich gegenüber der Abtriebswelle erstreckt, eine Trägerstruktur (319,320,321) mit einem Universallager (316) zum Halten des mittleren Teils des Stabes und eine Behälterhalte-Einrichtung (350,352) zum Halten des Behälters (7), die mit dem freien Ende des Stabes in Eingriff gebracht werden kann, wobei die Trägerstruktur (319,320,321) durch die Rahmenstruktur über eine stoßabsorbierende Einrichtung (326) gestützt wird und die Behälterhalte-Einrichtung (350,352) in ihr aufgehängt ist, und eine Einrichtung (330,332), die eine Umdrehung der Behälterhalte-Einrichtung (350,352) nur dann gestattet, wenn ein Schütteln gewünscht wird, welche Einrichtung einen Elektromagneten (330) mit einem Anker (332) umfaßt, der mit dem exzentrischen Gewichtselement (322) in Eingriff gebracht werden kann.

2. Gerät nach Anspruch 1, worin die Behälterhalteöffnung (710) eine Größe und Konfiguration hat, die geeignet ist den Behälter (7) durch einen Umfangsrandbereich der Öffnung (710) zu stützen; die Behälterzufuhr-Einrichtung (100) über der ersten Position (P1) der Drehscheibe (700) angeordnet ist und eine Zylinder-Einrichtung (102) mit einem zur stapelweisen Aufnahme von mehreren Behältern (7) geeigneten Querschnitt, eine Klappen-Einrichtung (104) im unteren Bereich der Zylinder-Einrichtung (102) zum normalerweise Schließen des unteren Bereiches des Zylinders (102) und Halten der Behälter (7) darin, ein erstes Plattenelement (110) und eine erste Hebe-Einrichtung (112,114) zum vertikalen Bewegen des ersten Plattenelements (110) durch die Behälterhalteöffnung (710) der Drehscheibe (700) an der ersten Position (P1) umfaßt, wobei das erste Plattenelement (110) so gestaltet ist, daß es beim Anheben durch die erste Hebe-Einrichtung (112, 114) die Klappen-Einrichtung (104) öffnet und die Unterseite des untersten Behälters (7) in dem Zylinder (102) stützt und beim Absenken durch die erste Hebe-Einrichtung (112,114) den untersten Behälter (7) von dem Zylinder (102) trägt und in die Behälterhalteöffnung (710) der Drehscheibe (700) an der ersten Position (P1) einsetzt.

3. Gerät nach Anspruch 1 oder 2, worin eine vertikale Einrichtung (800) an einer der ersten Position (P1) benachbarten zweiten Position (P2) vorgesehen ist zum Heben des Behälters (7), der durch die Behälterhalteöffnung (710) gestützt und von der ersten Position (P1) her bewegt wird, zu der Vorbehandlungs-Einrichtung (200), die auf dem oberen Deck (60) der Rahmenstruktur angeordnet ist, und zur Rückführung des Behälters (7) nach der dort stattgefundenen Vorbehandlung zu der Behälterhalteöffnung (710).

4. Gerät nach Anspruch 3, worin die vertikale Einrichtung (800) ein zweites Plattenelement (110) und eine zweite Hebe-Einrichtung (802,804) zum vertikalen Bewegen des Plattenelements (110) durch die Behälterhalteöffnung (710) der Drehscheibe (700) an der zweiten Position (P2) umfaßt.

5. Gerät nach irgendeinem der Ansprüche 1-4, worin die Vibrations-Einrichtung (300) an einer dritten Position (P3) der Drehscheibe (700) angeordnet ist und die Informationsaufnahme-Einrichtung (400) und die Rückführungs-Einrichtung (500) nacheinander an den letzten beiden Positionen der Drehscheibe (700) angeordnet sind, wobei die Umfangs-Drehgeschwindigkeit der Drehscheibe (700) so bestimmt wird, daß während der Rotation entlang eines Umfangsabstandes der Drehscheibe (700) zwischen der dritten Position (P3) und einer stromaufwärts von den letzten beiden Positionen liegenden Position ausreichend Zeit bleibt, um den gewünschten Grad der immunologischen Reaktion zu erzielen.

**Revendications**

1. Un appareil d'analyse immunologique automatique comprenant

des moyens d'alimentation de récipients (100) pour alimenter successivement des récipients (7), chacun d'eux comprenant une pluralité de puits,

des moyens de traitement préalable (200) pour alimenter un réactif immunologique d'agglutination contenant un antigène ou un anticorps et des échantillons contenant, éventuellement, des anticorps ou des antigènes dans les puits de chaque récipient (7),

des moyens de vibration (300) pour faire vibrer ledit récipient (7) de manière à agiter le réactif et les échantillons dans lesdits puits,

des moyens de maintien pour maintenir la stabilité dudit récipient (7) pendant un temps suffisamment long pour qu'une réaction antigène-anticorps entre l'échantillon et le réactif puisse se produire dans lesdits puits pendant le transport dudit récipient (7),

des moyens de détection de données (400) pour obtenir des images du processus d'agglutination dans lesdits puits dudit récipient (7), résultant de la réaction antigène-anticorps,

des moyens d'évaluation (600) pour évaluer les images d'agglutination en fonction des données fournies par lesdits moyens de détection de données (400), et

des moyens de récupération (500) pour récupérer ledit récipient (7) lorsque l'évaluation est terminée,

caractérisé en ce qu'il est prévu une structure formant châssis comprenant un plateau supérieur (60), un plateau intermédiaire (50) et un plateau inférieur, lesdits moyens de traitement préalable (200) étant disposés sur ledit plateau supérieur (60) de ladite structure formant châssis;

en ce que lesdits moyens de maintien comprennent un disque rotatif (700), monté à rotation sur ledit plateau intermédiaire (50) de ladite structure formant châssis et comprennent une pluralité d'ouvertures de support de récipient espacées équiangulairement (710), disposées sur sa périphérie, et des premiers moyens d'entraînement (702, 704, 706) pour faire tourner ledit disque rotatif (700) de façon intermittente à partir d'une première position (P1) jusqu'à une dernière position (P5) de manière à effectuer l'analyse d'un récipient (7) pendant le transport dudit récipient (7) de ladite première position (P1) jusqu'à ladite dernière position (P5) en une révolution complète dudit disque rotatif (700); et

en ce que lesdits moyens de vibration (300) comprennent un moteur électrique (318) pourvu d'un arbre de sortie sur lequel est monté un élément de contrepoids excentrique (322) et une tige (310) se prolongeant à l'opposé dudit arbre de sortie, une structure de support (319, 320, 321) comprenant un palier à cardan (316) pour supporter la partie centrale de ladite tige, et des moyens de maintien de récipient (350, 352) pouvant venir en prise sur l'extrémité libre de ladite tige pour maintenir ledit récipient (7), ladite structure formant support (319, 320, 321) étant supportée par ladite structure de châssis par l'intermédiaire de moyens formant amortisseur (326) et assurant la suspension desdits moyens de maintien de récipient (350, 352), et des moyens (330, 332) pour permettre auxdits moyens de maintien de récipient (350, 352) de ne tourner que lorsqu'une agitation est souhaitée, lesdits moyens comprenant un solénoïde (330) muni d'un plongeur (332) susceptible de venir en prise sur ledit élément formant contrepoids excentrique (322).

2. L'appareil selon la revendication 1, dans lequel les dimensions et la configuration de ladite ouverture de support de récipient (710) supportent de manière adéquate ledit récipient (7) par une partie de bord périphérique de ladite ouverture (710); lesdits moyens d'alimentation de récipient (100) sont disposés au dessus de ladite première position (P1) dudit disque rotatif (700) et comprennent des moyens en forme de cylindre (102) dont la section en coupe transversale est apte à recevoir une pluralité desdits récipients (7) empilés, des moyens en forme de volet obturateur (104) étant prévus dans une partie de fond desdits moyens formant cylindre (102) pour fermer normalement ladite partie de fond dudit cylindre (102) afin de maintenir lesdits récipients (7) à l'intérieur, un premier élément en forme de plaque (110) et des premiers moyens d'élévation (112, 114) pour déplacer verticalement ledit premier élément en forme de plaque (110) à travers ladite ouverture de support de récipient (710) dudit disque rotatif (700) dans ladite première position (P1), ledit premier élément en forme de plaque (110) étant agencé pour ouvrir lesdits moyens en forme de volet obturateur (104) et supporter la surface inférieure du récipient (7) le plus bas dans ledit cylindre (102) lorsqu'il est élevé par lesdits premiers moyens d'élévation (112, 114) et pour transporter le récipient (7) le plus bas dudit cylindre (102) et le placer sur ladite ouverture de support de récipient (710) dudit disque rotatif (700) dans lesdites premières positions (P1) lorsqu'il est abaissé par lesdits premiers moyens d'élévation (112, 114).

3. L'appareil selon la revendication 1 ou 2, dans lequel il est prévu des moyens verticaux (800) dans une seconde position (P2) voisine de ladite première position (P1) pour élever ledit récipient (7) supporté par ladite ouverture de support de récipient (710) et le déplacer de ladite première position (P1) jusqu'auxdits moyens de traitement préalables (200) disposés sur ledit plateau supérieur (60) de ladite structure formant châssis et, après le traitement préalable, ramener ensuite ledit récipient (7) dans ladite ouverture de support de récipient (710).

4. L'appareil selon la revendication 3, dans lequel lesdits moyens verticaux (800) comprennent un second élément en forme de plaque (110) et des seconds moyens d'élévation (802, 804) pour déplacer verticalement ledit élément en forme de plaque (110) à travers ladite ouverture de support de récipient (710) dudit disque rotatif (700) dans ladite deuxième position (P2).

5. L'appareil selon l'une quelconque des revendications 1 à 4, dans lequel lesdits moyens de vibration (300) sont disposés dans une troisième position (P3) dudit disque rotatif (700) et lesdits moyens de détection de données (400) et lesdits moyens de récupération (500) sont disposés dans les deux dernières positions respectives successives dudit disque rotatif (700), et dans lequel la vitesse de rotation périphérique dudit disque rotatif (700) est déterminée de manière que le temps soit suffisant pour obtenir le degré de réaction immunologique souhaité pendant la rotation le long d'une distance périphérique dudit disque rotatif (700) entre ladite troisième position (P3) et une position en amont desdites deux dernières positions.

*FIG. 1*

*FIG. 2*

# FIG. 3

# FIG. 4

|   | I | II | III | IV | V | VI |
|---|---|---|---|---|---|---|
| A | 51    3 | 49    4 | 48    2 | 47    7 | 45    3 | 44    9 |
| B | 49    7 | 47    8 | 45    9 | 43    11 | 35    30 | 32    28 |
| C | 48    4 | 47    3 | 42    13 | 32    34 | 26    32 | 27    12 |
| D | 47    7 | 44    10 | 32    37 | 25    36 | 24    11 | 30    0 |
| E | 47    3 | 35    33 | 28    27 | 26    20 | 21    12 | 28    0 |
| F |   |   | 36 | 25 |   |   |

## FIG. 5

|   |   |   |   |   |   |   |   |   |   |   |   |   |   |   |   |   |   |   |   |   |   |
|---|---|---|---|---|---|---|---|---|---|---|---|---|---|---|---|---|---|---|---|---|---|
| a |   |   |   |   |   |   |   |   |   |   |   |   |   |   |   |   |   |   |   |   |   |
| b |   |   |   |   |   |   |   |   |   |   |   |   |   |   |   |   |   |   |   |   |   |
| c |   |   | 2 | 2 | 4 | 5 | 6 | 11 | 10 | 6 | 8 | 7 | 3 | 5 | 2 | 1 |   |   |   |   |   |
| d |   | 3 | 4 | 2 | 7 | 3 | 9 | 33 | 35 | 38 | 38 | 35 | 34 | 7 | 4 | 9 | 3 | 5 |   |   |   |
| e |   | 7 | 8 | 9 | 11 | 30 | 28 | 27 | 26 | 29 | 27 | 27 | 29 | 28 | 30 | 12 | 8 | 7 | 6 |   |   |
| f | 3 | 4 | 3 | 13 | 34 | 32 | 12 | 16 | 17 | 15 | 16 | 18 | 20 | 17 | 32 | 33 | 13 | 4 | 2 | 4 |   |
| g | 2 | 7 | 10 | 37 | 36 | 11 | 0 | 2 | 4 | 5 | 2 | 7 | 4 | 13 | 17 | 35 | 37 | 11 | 8 | 4 |   |
| h | 4 | 6 | 3 | 33 | 27 | 20 | 12 | 0 | 4 | 8 | 4 | 0 | 4 | 7 | 10 | 11 | 20 | 26 | 34 | 3 | 7 | 3 |
| i |   |   |   |   |   |   |   |   |   |   |   |   |   |   |   |   |   |   |   |   |   |
| j |   |   |   |   |   |   |   |   |   |   |   |   |   |   |   |   |   |   |   |   |   |
| k |   |   |   |   |   |   |   |   |   |   |   |   |   |   |   |   |   |   |   |   |   |
| l |   |   |   |   |   |   |   |   |   |   |   |   |   |   |   |   |   |   |   |   |   |

## FIG. 6

600

JUDGEMENT — A

100 CONTAINER SUPPLY — P1

200 SAMPLE + REAGENT SUPPLY — P2

300 AGITATION — P3

400 DETECTION — P4

500 CONTAINER RECOVERY — P5

|← REACTION TIME (T) →|

# FIG. 7

# FIG. 8

# FIG. 9

# FIG. 10

*FIG. 11*

*FIG. 12*

# FIG. 13

# FIG. 14

# FIG. 15

FIG. 16

FIG. 17a

FIG. 17b

FIG. 18a

FIG. 18b

FIG. 19

FIG. 20

FIG. 21

FIG. 22

# FIG. 23

## FIG. 24